# EUROPÄISCHE PATENTSCHRIFT

(11) **EP 1 263 703 B1**
(45) Veröffentlichungstag und Bekanntmachung des Hinweises auf die Patenterteilung: **25.04.2007**
(21) Anmeldenummer: 00981268.6
(22) Anmeldetag: 17.11.2000
(51) Int. Cl.: C07C 37/20, B01J 31/40, C07C 39/16

(54) **INBETRIEBNAHMEVERFAHREN EINES HERSTELLUNGSVERFAHRENS VON 2,2-BIS (4-HYDROXYPHENYL)PROPAN**
INITIATION METHOD OF A METHOD FOR PRODUCING 2,2-BIS(4-HYDROXYPHENYL)PROPANE
PROCEDE DE MISE EN OEUVRE D'UN PROCEDE DE PREPARATION DE 2,2-BIS(4-HYDROXYPHENYL)PROPANE

(30) Priorität: 30.11.1999 DE 19957602
(43) Veröffentlichungstag der Anmeldung: 11.12.2002
(73) Patentinhaber: Bayer MaterialScience AG, 51368 Leverkusen (DE)
(72) Erfinder: BÖDIGER, Michael, League City, TX 77573 (US); NEUMANN, Rainer, 47803 Krefeld (DE); HEYDENREICH, Frieder, 40593 Düsseldorf (DE); PREIN, Michael, B-2930 Brasschaat (BE); FOFONKA, Hans-Ludwig, 47877 Willich (DE)
(86) Internationale Anmeldenummer: PCT/EP2000/011465
(87) Internationale Veröffentlichungsnummer: WO 2001/040155

(56) Entgegenhaltungen:
- WO-A-99/33777
- US-A- 5 502 016

## Beschreibung

Die Anmeldung betrifft die Inbetriebnahme eines Verfahrens zur Herstellung von Bisphenolen.

Die Herstellung von Bisphenolen ist bekannt und erfolgt im allgemeinen durch säurekatalysierte Umsetzung von Phenolen mit Carbonylverbindungen. Die Reaktion wird im allgemeinen in Festbett- oder Wirbelbettreaktoren wie auch in Reaktivkolonnen durchgeführt.

Als Katalysatoren werden bevorzugt vernetzte sulfonierte Polystyrolharze (saure Ionentauscher) zum Einsatz gebracht. Diese Ionentauscher können gegebenenfalls durch kovalent oder ionisch gebundene Cokatalysatoren chemisch modifiziert sein und sind makroporös oder gelförmig (US-4,191, 843; US-3,037,052).

In US-A-5 502 016 wird die Behandlung von Ionenaustauschen mit einem Gemisch aus Phenol und geringen Mengen Aceton, mit anschließender Herstellung von Bisphenol A bei erhöhter Acetonmenge beschrieben. Eine Aufrechterhaltung der Leistung und Stabilität des Katalysators über eine längeren Zeitraum durch kontrollierte Erhöhung der Acetons und des Mengendurchsatz wird in US-A 5 502 016 nicht offenbart.

Für eine effiziente und ökonomische Herstellung von z.B. 2,2-Bis(4-hydroxyphenyl)propan (BPA) durch Umsetzung von Phenol mit Aceton sollen einerseits durch geeignete Wahl der Reaktionsbedingungen möglichst hohe Acetonumsätze bei gleichzeitig hoher Selektivät für p,p-BPA erreicht werden, andererseits ist entscheidend, daß der Katalysator (Ionentauscher) seine Aktivität über einen langen Zeitraum aufrecht erhält, um den aufwendigen Katalysatoraustausch zu vermeiden.

In der Literatur wird daher der Vorbereitung und Reaktivierung von Katalysatoren in der BPA Herstellung verstärkte Aufmerksamkeit zuteil.

So beschreibt die EP-A 765 685, daß die vorherige Wäsche und Entwässerung von kommerziellen Ionentauschern zu höherer Produktreinheit führt. Die EP-A 680 786 lehrt, das desaktivierte Ionentauscher durch Spülen mit wasserfreiem Phenol bei höheren Temperaturen reaktiviert werden können.

Aufgabe ist eine optimierte Inbetriebnahme eines kontinuierlichen Verfahrens zur Herstellung von BPA durch Umsetzung von Phenol mit Aceton in Gegenwart von sulfonierten vernetzten Polystyrolharzen.

Es wurde nun überraschend gefunden, daß einerseits die Gesamtmenge an Reaktionsmischung bei der Inbetriebnahme, und andererseits zusätzlich auch die Zusammensetzung der Reaktionsmischung Einfluß auf die katalytische Leistung und Lebensdauer des Katalysatorsystems hat.

Gegenstand der Anmeldung ist daher ein Verfahren zur Inbetriebnahme eines Herstellungsverfahrens von 2,2-Bis(4-hydroxyphenyl)propans durch Umsetzug von Phenol mit Aceton in Gegenwart von sulfonierten vernetzten Polystyrolharzen, das dadurch gekennzeichnet ist, dass
a) die Inbetriebnahme mit einer gegenüber dem optimalen Dauerbetriebszustand verringerten Acetonkonzentration und erhöhten Phenolkonzentration unter verringertem Mengendurchsatz erfolgt und
b) unter Temperaturkontrolle anschließend die Eduktmenge und der Acetongehalt des Reaktorzulaufs stufenweise oder kontinuierlich erhöht wird bis der Dauerbetriebszustand erreicht ist.
wobei der Mengendurchsatz bei der Inbetriebnahme gegenüber dem im Dauerbetrieb optimalen Mengendurchsatz um 40 % oder mehr reduziert wird und die Acetonkonzentration bei der Inbetriebnahme gegenüber der im Dauerbetrieb optimalen Konzentration um 30 % oder mehr erniedrigt wird.

Prinzipiell kann die Kondensationsreaktion zwischen Phenol und Aceton mit Acetonkonzentration von bis zu 15 Gew.-% durchgeführt werden. Während hohe Acetonkonzentrationen zu einer erhöhten BPA-Produktion und somit zu erhöhten Raum-Zeit-Ausbeuten führen, kann die freiwerdende Wärme bei der Kondensationsreaktion zu technischen Limitationen führen. Außerdem führt eine erhöhte Acetonkonzentration zu verringerten Selektivitäten in der Reaktion.

Die Acetonkonzentration wird beim ersten Kontakt des Reaktionsgemischs mit dem Katalysatorsystem gegenüber der im Dauerbetrieb bevorzugten

Acetonkonzentration um mindestens 30 %, bevorzugt um mindestens 45 % reduziert. Die Gesamtmenge an Reaktionsmischung wird gegenüber der im Dauerbetrieb bevorzugten Menge um mindestens 40 %, bevorzugt mindestens 60 % reduziert.

Die Temperaturen am Einlaß liegen vorzugsweise bei 45 bis 80°C, bevorzugt bei 50 bis 65°C.

Durch Temperaturmeßstellen am Reaktor wird die Temperaturerhöhung im Reaktor verfolgt. Hierbei sollen für jede Meßstelle Temperaturen von 110°C, bevorzugt von 100°C, besonders bevorzugt von 90°C nicht überschritten werden. Das Verfahren wird vorzugsweise so geführt, dass eine Temperaturspitze den Reaktor durchläuft. Wenn die Temperaturspitze den Auslaß des Reaktors erreicht, wird dann die Acetonmenge im Zulauf kontinuierlich oder stufenweise bis zu der im Dauerbetrieb bevorzugte Acetonkonzentration erhöht. Gleichzeitig oder bevorzugt anschließend wird die Gesamtmenge am Zulauf des Festbettreaktors stufenweise oder kontinuierlich auf die im Dauerbetrieb bevorzugte Gesamtmenge erhöht. Anschließend wird gegebenenfalls die Reaktionstemperatur auf die Dauerbetriebstemperatur eingestellt.

Für den Dauerbetrieb zur Herstellung von BPA werden Phenol und Aceton vorzugsweise im Verhältnisse von > 5 : 1, bevorzugt > 10 :1 eingesetzt. Dabei haben sich Acetonkonzentrationen im Reaktionsgemisch von 2,0 Gew.-% bis 15,0 Gew.-%, insbesondere 3,5 Gew.-% bis 5,5 Gew.-% als günstig erwiesen. Die Reaktion erfolgt im Dauerbetrieb im allgemeinen bei 45 bis 110°C, bevorzugt bei 50 bis 80°C.

Der Gehalt an gelöstem Sauerstoff in der Reaktionsmischung für die Inbetriebnahme und im Dauerbetrieb beträgt vorzugsweise weniger als 1 ppm, bevorzugt weniger als 100 ppb. Der Gehalt an gelösten oder ungelösten Metallionen in der Reaktionsmischung für die Inbetriebnahme und im Dauerbetrieb ist vorzugsweise nicht größer als 1 ppm, bevorzugt nicht größer als 0,5 ppm für Fe, Co, Ni, Mo, Cr, Cu als individuelle Komponenten und vorzugsweise nicht größer als 10 ppm, bevorzugt nicht größer als 1 ppm für die Summe der genannten Metalle.

Als Reaktor wird bevorzugt ein Schichtbett- oder Wirbelbettreaktor, die auf- oder abwärts durchflossen werden und insbesondere ein kontinuierlich von oben nach unten durchströmter Schichtbettreaktor eingesetzt.

Das erfindungsgemäße Inbetriebnahme-Verfahren ist sowohl zum ersten Anfahren eines Reaktionssystems nach Wechsel des Katalysators wie auch zur Wiederinbetriebnahme beispielsweise nach zwischenzeitlicher Abstellung geeignet.

Die Inbetriebnahme mit verringerten Aceton- und erhöhten Phenolkonzentrationen ist vorteilhaft sowohl beim Einsatz von reinen Aceton/Phenolmischungen wie auch beim technisch bevorzugten Betrieb von Reaktionseinheiten mit rückgeführten Kreislaufströmen enthaltend Phenol, Aceton, BPA, BPA-Isomere und Nebenprodukte und optional Wasser.

Der Vorteil des erfindungsgemäßen Vorgehens besteht darin, daß durch die Kontrolle von Acetonkonzentration, Menge und Temperatur eine irreversible Schädigung des Katalysatorharzes vermieden wird. Eine derartige Schädigung tritt bei der Inbetriebnahme des Katalysatorsystems mit den im Dauerbetrieb bevorzugten Acetonkonzentrationen auf und zeigt sich in mechanischer Beschädigung der Katalysatorperlen und gefärbten Belegungen auf den Oberflächen der Katalysatorperlen. Diese Schäden führen zu verringerten Aktivitäten und Selektivitäten des Katalysators im Dauereinsatz und aufgrund einer Veränderung der Kompressibilität zu einem erhöhten Druckaufbau im Dauerbetrieb. Daneben ergibt sich auch eine vermehrte Farbbildung bei der Kondensationsreaktion von Phenol mit Aceton. Durch den Ausschluß von Sauerstoff und die Kontrolle des Metallionenkonzentration in der Reaktionslösung wird sichergestellt, daß der Ionentauscher nicht durch Belegung mit ionischen Bestandteilen in seiner Aktivität beeinträchtigt wird und daß durch den Effekt von redoxaktiven Metallbestandteilen und Sauerstoff keine Abbauerscheinungen an der organischen Matrix des Ionentauschers auftreten. Somit kann bei erfindungsgemäßem Vorgehen die Ausbeute und Qualität von BPA in einer technisch betriebenen BPA-Anlage erhöht werden und der aufwendige Austausch von Katalysatorbetten In Folge von Desaktivierung verringert werden.

Die nachfolgenden Beispiele dienen zur Erläuterung der Erfindung. Die Erfindung ist nicht auf die Beispiele limitiert.

### Beispiel 1

Zur technischen Herstellung von BPA wird sulfoniertes, vernetztes Polystyrolharz (Lewatit SC 104, Bayer AG) eingesetzt. Das Reaktionssystem wird mit einer entwässerten Mutterlauge aus einer Apparatur zur Abtrennung von BPA-Phenol-Adduktkristallen betrieben, der vor Eintritt in den Reaktor mit Phenol und Aceton ergänzt wird. Die typische und bezüglich des Acetonsgehalts im Dauerbetrieb optimale Zusammensetzung beträgt:
Phenol: 81,0 %
Aceton: 4,0 %
p,p-BPA: 9,0 %
Isomere und höhere Kondensate: 6,0 %
Wasser: < 0,1 %

Der Durchsatz im Dauerbetrieb beträgt 0,3 m³ Reaktionslösung / m³ Katalysatorvolumen * h bei einer Eintrittstemperatur von 58 °C.
Die Inbetriebnahme des Reaktionssystems erfolgt nach einem Katalysatorwechsel in folgender Weise: Der phenolfeuchte Katalysator wir als Suspension in Phenol (70 Vol.-% Feststoffanteil) bei 60 °C in den Reaktor (adiabatisch betriebener, abwärts durchflossener Festbettreaktor) überführt. Überstehendes Phenol wird abgelassen, das Katalysatorbett einmal mit Phenol (100 Vol-%) gespült und nach Ablassen des Phenols von unten im Gegenstrom bis zur Oberkante des Katalysatorbetts mit Phenol befüllt. Anschließend wird das Katalysatorbett bei 58 °C mit Reaktionslösung beaufschlagt. Durch Zumischen von Phenol (100 Gew.-%) zu der im Dauerbetrieb üblichen Reaktionsmischung wird hierbei eine Reaktionsmischung der folgenden Zusammensetzung eingestellt:
Phenol: (90,5 %)
Aceton: 2.0 %
p,p-BPA: 4,5 %
Isomere und höhere Kondensate: 3,0 %
Wasser: < 0,1 %

Das Reaktionssystem wird mit 0,06 m³ Reaktionslösung / m³ Katalysatorvolumen * h (entsprechend 20 % des im Dauerbetrieb optimalen Durchsatzes) kontinuierlich durchflossen. Mittels Temperaturfühlern auf 20, 40, 60, 80 und 100 % der Katalysatorbetthöhe wird der Temperaturverlauf entlang des Katalysatorbetts verfolgt. Es tritt eine Temperaturspitze von 85 °C auf, die sich entlang des Katalysatorbetts fortpflanzt und nach 15 h am Auslaß registriert wird. Zu diesem Zeitpunkt wird die Reaktionsmischung am Reaktoreintritt auf die oben angegebenen, für den Dauerbetrieb optimalen Werte (4.0 % Aceton) eingestellt. Anschließend wird die Durchsatzmenge im Verlauf von 40 h kontinuierlich von 0,06 m³ Reaktionslösung / m³ Katalysatorvolumen * h auf die für den Dauerbetrieb optimale Menge von 0,3 m³ Reaktionslösung / m³ Katalysatorvolumen * h erhöht. Anschließend werden die eingestellten Werte im Dauerbetrieb beibehalten.

Ein auf derartige Weise in Betrieb genommener Katalysator zeigt im Dauerbetrieb über 60 Tage eine durchschnittlichen Acetonumsatz von 91 % und eine durchschnittliche Selektivität bezüglich der Bildung von p,p-BPA aus Aceton von 93.5 %. Der durchschnittliche Druckaufbau entlang des Katalysatorbetts betrug 0,2 bar/m. Katalysatorproben aus der Oberschicht des Reaktors zeigten einen durchschnittlichen Anteil an gebrochenen Katalysatorperlen von 5 %.

### Vergleichsbeispiel 2

Die Inbetriebnahme des Katalysator geschah wie in Beispiel 1 beschrieben nur erfolgte die Beaufschlagung direkt mit der für den Dauerbetrieb optimalen Reaktionsmischung (4.0 % Aceton) bei einem Durchsatz von 0,1 m³ Reaktionslösung/m³ Katalysatorvolumen * h , der anschließend über 5 h auf 0,3 m³ Reaktionslösung/m³ Katalysatorvolumen * h erhöht wurde. Hierbei wurden Temperaturspitzen von 115 °C im Reaktorbett gemessen.

Ein auf derartige Weise in Betrieb genommener Katalysator zeigt im Dauerbetrieb über 60 Tage eine durchschnittlichen Acetonumsatz von 87 % und eine durchschnittliche Selektivität bezüglich der Bildung von p,p-BPA aus Aceton von 92.2 %. Der durchschnittliche Druckaufbau entlang des Katalysatorbetts betrug 0,3 bar/m. Katalysatorproben aus der Oberschicht des Reaktors zeigten einen durchschnittlichen Anteil an gebrochenen Katalysatorperlen von 15 %. Die Katalysatorperlen waren mit einer schwarzen Oberflächenbelegung bedeckt. Gegenüber den Ergebnissen aus Beispiel 1 war die Farbe der Reaktionslösung am Reaktoraustritt um durchschnittlich 5 Hazen erhöht.

## Patentansprüche

1. Verfahren zur Inbetriebnahme eines Herstellungsverfahrens von 2,2-Bis (4-hydroxyphenly)Propans durch Umsetzung von Phenol mit Aceton in Gegenwart von sulfonierten vernetzten Polystyrolharzen, **dadurch gekennzeichnet ist, dass**
a) die Inbetriebnahme mit einer gegenüber dem optimalen Dauerbetriebszustand verringerten Acetonkonzentration und erhöhten Phenolkonzentration unter verringertem Mengendurchsatz erfolgt und
b) unter Temperaturkontrolle anschließend die Eduktmenge und der Acetongehalt des Reaktorzulaufs stufenweise oder kontinuierlich erhöht wird bis der Dauerbetriebszustand erreicht ist,
wobei der Mengendurchsatz bei der Inbetriebnahme gegenüber dem im Dauerbetrieb optimalen Mengendurchsatz um 40 % oder mehr reduziert wird und die Acetonkonzentration bei der Inbetriebnahme gegenüber der im Dauerbetrieb optimalen Konzentration um 30 % oder mehr erniedrigt wird.

2. Verfahren nach Anspruch 1, **dadurch gekennzeichnet, dass** die im Dauerbereich optimale Acetonkonzentration 2,0 bis 15,0 Gew.-% beträgt.

## Claims

1. Process for the commissioning of a process for the production of 2,2-bis(4-hydroxyphenyl)propane by reacting phenol with acetone in the presence of sulfonated cross-linked polystyrene resins,
**characterised in that**
a) the commissioning takes place with an acetone concentration reduced below, and a phenol concentration increased above, that of the optimal sustained operation state, with a reduced quantitative throughput, and
b) with temperature control, the reactant quantity and the acetone content of the reactor feed are subsequently increased in step-wise or continuous manner until the sustained operating state is reached,
wherein on commissioning the quantitative throughput is reduced by 40% or more below the optimal quantitative throughput in sustained operation, and on commissioning the acetone concentration is lowered by 30% or more below the optimal concentration in sustained operation.

2. Process according to claim 1, **characterised in that** the optimal acetone concentration in sustained operation is from 2.0 to 15.0 wt.%.

## Revendications

1. Procédé de mise en service d'un procédé de fabrication de 2,2-bis(4-hydroxyphényl)propane par réaction du phénol avec l'acétone en présence de résines de polystyrène sulfonées réticulées qui est **caractérisé en ce que**
a) la mise en service se fait avec une concentration en acétone diminuée et une concentration en phénol augmentée par rapport à l'état optimal en fonctionnement continu avec un débit diminué et
b) sous contrôle de la température, on augmente ensuite par étape ou de manière continue la quantité d'éduit et la teneur en acétone de l'alimentation du réacteur jusqu'à ce que l'état de fonctionnement continu soit atteint,
le débit à la mise en service étant'réduit de 40 % ou plus vis-à-vis du débit optimal en fonctionnement continu et la concentration en acétone lors de la mise en service étant abaissée de 30 % ou plus vis-à-vis de la concentration optimale en fonctionnement continu.

2. Procédé suivant la revendication 1, **caractérisé en ce que** la concentration en acétone optimale en fonctionnement continu est de 2,0 à 15,0 % en poids.
